# EUROPEAN PATENT SPECIFICATION

(11) **EP 2 666 364 B1**
(45) Date of publication and mention of the grant of the patent: **03.04.2019**
(21) Application number: 12736454.5
(22) Date of filing: 20.01.2012
(51) Int. Cl.: A61K 31/19, A61K 31/232, A61K 36/67, A61K 36/9068, A61K 9/00, A61K 31/05, A61K 31/11, A61K 31/20, A61K 31/4525, A23K 20/105, A23K 20/158, A23K 20/10, A23K 50/00, A23K 50/75

(54) **ADDITIVES FOR ANIMAL FOOD**
ZUSATZSTOFFE FÜR TIERFUTTER
ADDITIFS POUR L'ALIMENTATION ANIMALE

(30) Priority: 21.01.2011 ES 201130070
(43) Date of publication of application: 27.11.2013
(73) Proprietor: Norel, S.A., 28007 Madrid (ES)
(72) Inventor: PABLOS PÉREZ, Enrique, E-28007 Madrid (ES)
(74) Representative: Manuel Illescas y Asociados, S.L.U.
(86) International application number: PCT/ES2012/070029
(87) International publication number: WO 2012/098282

(56) References cited:
- WO-A1-2008/155536
- WO-A2-2004/091307
- GB-A- 2 466 041
- US-A1- 2007 116 852
- US-A1- 2009 004 308
- C. FERNANDEZ-RUBIO ET AL: "Butyric acid-based feed additives help protect broiler chickens from Salmonella Enteritidis infection", POULTRY SCIENCE, vol. 88, no. 5, 1 May 2009 (2009-05-01), pages 943-948, XP055317859, ISSN: 0032-5791, DOI: 10.3382/ps.2008-00484
- K. A. HAMMER ET AL: "Antimicrobial activity of essential oils and other plant extracts", JOURNAL OF APPLIED MICROBIOLOGY, vol. 86, no. 6, 1 June 1999 (1999-06-01), pages 985-990, XP055039370, ISSN: 1364-5072, DOI: 10.1046/j.1365-2672.1999.00780.x
- WINDISCH W ET AL: "Use of phytogenic products as feed additives for swine and poultry", JOURNAL OF ANIMAL SCIENCE, AMERICAN SOCIETY OF ANIMAL SCIENCE, US, vol. 86, no. 14 Suppl, 1 April 2008 (2008-04-01), pages E140-E148, XP002657131, ISSN: 0021-8812, DOI: 10.2527/JAS.2007-0459 [retrieved on 2007-12-11]
- "Ginger", , Retrieved from the Internet: URL:http://en.wikipedia.org/wiki/ginger [retrieved on 2018-07-09]
- "Black pepper", , Retrieved from the Internet: URL:http://en.wikipedia.org/wiki/black_pep per [retrieved on 2018-07-09]

## Description

### FIELD OF THE INVENTION

The present invention refers to additives for animal feed based on sodium butyrate combined with the essential oils of ginger and the essential oil piperine together coated with vegetable fats and/or oils comprising palm stearin. These additives are used as promoters or stimulants of animal growth, modulators of the immune response and as bactericides. Therefore, the present invention is in the field of animal production and more specifically in the fields of animal feed and health.

### STATE OF THE ART

The trend in animal feed in Europe in recent years has been the search for a diet, which not only covers the feeding needs of the animals but also reinforces their health and reduces pathological problems without using medicines, to improve production and economic yields of the farmer. One of the ways of controlling the heath and immunity of the animal is by means of controlling intestinal flora.

The use of additives has been common practice in animal feeding with the aim of improving production yields, improving health and thus achieving more efficient utilisation of the food. Because of the prohibition of the use of antibiotics as growth promoting substances in the European Union, (directive 1831/2003/CEE), additives have been sought that often demonstrate improved production compared even to the antibiotics themselves (Dipeolu et al 2005). Various studies carried out in recent years have shown that organic acids are a good alternative. Their mode of action lies, on the one hand, in reducing the pH in the stomach, which limits development of pathogens and helps in protein digestion and, on the other hand, in the ability of some of these organic acids to enter bacteria and block their metabolism. In this sense, butyric acid, also known as butanoic acid, has been studied for several years and beneficial effects have been observed in several species: pigs, poultry, ruminants, etc. and even in fish. Together with acetic and propanoic acids, butyric acid belongs to the group of short-chain volatile fatty acids (VFA). It has been demonstrated that short-chain VFA can inhibit growth of the Enterobacteriaceae bacterial group (*Salmonella, Escherichia coli,* etc.), (Van Immerseel et al 2004). This inhibition occurs owing to the fact that the undissociated forms of the volatile fatty acids are able to cross bacterial membranes, interfere in bacterial metabolism and bring about their death. The acid anion also interferes with gene transcription of the bacteria, which prevents them from reproducing and causing infection. Butyric acid has a better diffusion coefficient than other VFAs, so it crosses the bacterial wall more easily than other acids.

Along with the antibacterial effect, butyric acid has additional effects that make it unique: for example, it stimulates pancreatic secretion (Katoh and Tsudo, 1984; Sano et al 1995), improves electrolyte absorption, reduces the incidence of diarrhoea, increases microvilli regeneration in the intestine and increases their length (Galfi and Bokori 1990, Lesson et al 2005) while also increasing the area of intestinal absorption giving rise to higher average weight of poultry and to better food conversion. Thus all these studies demonstrate that butyric acid is beneficial for the livestock farmer, both in terms of nutrition and in terms of health.

It should be pointed out that the free form of butyric acid is difficult to handle because of its high corrosivity and volatility. To solve this problem, various presentations of butyric acid have been developed: salts of butyric acid, salts of microencapsulated butyric acid and salts of butyric acid protected in a matrix of vegetable fats and/or oils, which protect part of the active ingredient and achieve slow release of this natural growth promoter, thus ensuring its growth potentiating and bactericidal action in the animal's digestive tract at the same time as providing the highest protected concentration possible, to include the product in a range of slow-release natural growth promoters. This protected acid salt is marketed under the name of Gustor BP-70 (Norel, SA, Spain); hereinafter in this invention it will be referred to as BP-70.

Partial protection of salts of butyric acid with vegetable oils and/or fats enables sodium butyrate to act effectively throughout the digestive tract of the animal, ensuring that the active ingredient reaches from the initial sections of the digestive tract to the most distal sections, not only acting as a natural growth promoter but also as a modulator of the immune response and a bactericide, reducing the possibility of infection by pathogenic bacteria. The effectiveness of Gustor BP-70 has been demonstrated in areas where the salts of unprotected butyric acid do not act (caecum/faeces) and in areas where these salts, in encapsulated form, have still not been able to act (crop), making it a complete and effective product in the fight against bacterial infections, especially against *Salmonella* Enteritidis, in bird nutrition (Fernandez-Rubio C et al 2009).

The above-referred document (Fernandez-Rubio C et al 2009) discloses that an additive consisting of a mixture of sodium butyrate partially protected with vegetable oils (commercial product Gustor XXI BP-70) showed a reduction of *Salmonella* Enteritidis infection in birds.

GB2466041A discloses coated granules comprising butyrate salt for use as an additive of animal feed. The granules furthermore comprise plant extracts, such as oregano oil or its components, thymol and carvacrol, cinnamaldehyde, ionone, artemisinin, eugenol, citrus extract, tannine extracts and grape extracts.

The document Hammer et al 1999 (K. A. Hammer et al, "Antimicrobial activity of essential oils and other plant extracts", Journal of Applied Microbiology, 1999, vol. 86, no. 6, pages 985 - 990) discloses tests on antimicrobial activity of essential oils and plant extracts against: *Acinetobacter baumanii, Aeromonas veronii* biogroup *sobria, Candida albicans, Enterococcus faecalis, Escherichia coli, Klebsiella pneumoniae, Pseudomonas aeruginosa, Salmonella enterica, Serratia marcescens* and *Staphylococcus aureus.* Essential oils of pepper and ginger are disclosed in this document.

The document Windisch et al 2008 (Windisch W et al, "Use of phytogenic products as feed additives for swine and poultry", Journal of Animal Science, 2008, vol. 86, no. 14 Suppl, pages E140 - E148) discloses phytogenic products as feed additives for swine and poultry. This document discloses that ginger and pepper have antioxidative properties.

Document US2007/116852A1 is related to the use of pepper as feed additive and discloses that the animals fed with pepper are less susceptible to illness.

The function of sodium butyrate as a bactericide has been mainly employed in the treatment of *Salmonella* Enteritidis, as this is one of the main pathogenic bacteria affecting animals and man. Among the pathogenic bacteria is the bacterium *Clostridium perfringens,* which causes necrotic enteritis as a consequence of the production of various toxins, resulting in extensive necrosis of the intestinal mucosa, mainly affecting chickens, turkeys, ducks and wild birds. *C. perfringens* occurs naturally in the intestine, proliferating under certain circumstances such as, for example, excess undegraded and undigested nutrients that appear more frequently with dietary changes, especially when animals receive poorly balanced diets or containing poor quality raw materials, poor immune response of the gut-associated lymphoid tissue (GALT) due to excess wear of the intestinal epithelium as a consequence of milling the feed, presence of mycotoxins, coccidia, etc., unbalancing of the intestinal microbial flora and other conditions. In 1972, with the release on the market of the coccidiostat Monensin, the appearance of cases of infection by *C. perfringens* appeared to stop but with the trend for removing antibiotics as growth promoters and for the addition of coccidiostats to the feed, there is a growing trend in the reappearance of this disease.

Therefore, to resolve this growing problem of the rise of infectious diseases in animals for fattening to be used for human consumption, and also to improve the growth of these animals, the present invention has developed a new additive for animal feed comprising sodium butyrate with the essential oils of ginger and the essential oil piperine together partially coated with vegetable fats and/or oils comprising palm stearin. This additive is able to inhibit bacterial growth, especially of Gram-positive bacteria and coccidia, and additionally improves the condition of the intestinal epithelium and improves the weight of treated animals. Partial coating of the active ingredients of this feed additive for animals, the salt of an organic acid and essential oils, with fats and/or oils of plant origin protects these active ingredients from digestion in the stomach, but enables them to be active throughout the gastrointestinal tract of animals because through this partial coating of the active ingredients, phases of the product with various amounts of fat (0% - 50% fat) can be produced that are digested and therefore released at different points of the gastrointestinal tract of the animals: the part unprotected by fat is released and has effect in the first sections of the gastrointestinal tract, up to the small intestine; the part protected by fat is not released until the fat starts to be digested by the action of pancreatic lipases. Given the slow digestion of the fat, *in vitro* studies have shown that part of the active ingredient can reach the most distal parts of the gastrointestinal tract, exercising its action there.

The feed additive described in the present invention is also able to potentiate the development of the intestinal epithelium, encouraging the growth of intestinal villi, which as a consequence of the necrotic enteritis induced in the animals described in the examples of the present invention are reduced in number and size. Furthermore, the increase in development of the intestinal epithelium encourages improved absorption of food, thereby causing an increase in growth and fattening of the animals feeding with the additive. The feed additive described in the present invention is also able to reduce the incidence of bacterial diseases such as necrotic enteritis or those caused by *Salmonella* Enteritidis, owing to the modulating action of the immune response and its bactericidal action against pathogens including *C. perfringens* and *S. enterica.* The combined use of the sodium butyrate and the essential oils of ginger and the essential oil piperine together partially coated with vegetable fats and/or oils comprising palm stearin is able to potentiate the physiological promoter effect of butyrate such as, for example as mentioned above, in the development of intestinal villi, in addition to potentiating its bactericidal effect.

The essential oils of ginger and the essential oil piperine are included in the combination with sodium butyrate. The main synergies of essential oils ginger and the essential oil piperine with sodium butyrate from a nutritional point of view are their action as stimulators of digestibility, because they encourage the balance and control of microbial flora; they are also important for their action as stimulators of immunity and antimicrobial and antioxidant properties.

Furthermore, in the present invention, the use of the combination of sodium butyrate and the essential oils of ginger and the essential oil piperine, together protected with oils and/or fats of plant origin comprising palm stearin, as a feed additive for animals is described, this feed additive acting as a natural growth promoter as well as bactericide and immunomodulator, able to reduce levels of pathogenic bacteria, specifically *C. perfringens* and *S. enterica,* and improving the immune response with which animals are protected from bacterial infections.

### DESCRIPTION OF THE INVENTION

### BRIEF DESCRIPTION OF THE INVENTION

The present invention describes a new additive for animal feed that acts as a natural promoter of animal growth and comprises sodium butyrate combined with essential oils of ginger and the essential oil piperine. This combination is partially protected from digestion in the stomach by a partial coating of vegetable fats and/or oils comprising palm stearin. This coating, being partial, does not prevent the action of the mentioned active ingredients throughout the whole intestinal tract, event to the most distal parts.

The term growth promoters or stimulators is given to those additives that form an integral part of animal diet and that perform the function of improving the daily weight gain of animals (DWG), as well as conversion of the ration consumed. In the present invention, growth promoters or stimulators can be administered by different ways, either by injection, in implants or as an additive in the feed of the animals. In the present invention, the effect of growth is measured as an increase in weight.

For the purpose of the present invention, the term organic acid refers to compounds containing one or more carboxylic groups (-COOH) in their formula, proton donors that can have various functional groups such as hydroxyacids, ketoacids, aromatic acids, heterocyclic compounds and also amides and lactones. The organic acids used in the present invention are preferably volatile fatty acids, preferably short-chain, and can be selected from: butyric acid, propionic acid, formic acid, lactic acid, citric acid, lauric acid, capric acid, caprylic acid, caproic acid, acetic acid and others. The proportions of the organic acid in the additive for animal feed described in the present invention may vary between 30% and 70% of the wet weight of the total product; preferably the proportion is 50%.

The salts of the organic acids used in the present invention are preferably the sodium salt, cupric salt, potassium salt, calcium salt and others. The proportions of sodium butyrate in the additive for animal feed described in the present invention may vary between 30% and 70% of the wet weight of the total product; preferably the proportion is 60%.

For the purpose of the present invention, the term active ingredient of plant origin refers to any material of plant origin to which appropriate activity can be attributed that is capable of exercising a beneficial effect on the organism to which it is applied. The active ingredients of plant origin used in the present invention are essential oils. For the purposes of the present invention, the term essential oil refers to a volatile organic substance or substances belonging to various classes of compounds, for example hydrocarbons, esters, alcohols, aldehydes, some acids, phenols and their derivatives, lactones, etc., all long-chain products from plant biosynthesis, called plant secondary metabolites. The term essential oil is also applied to similar synthetic substances, prepared from coal tar, and semi-synthetic substances prepared from the natural essential oils. The essential oils used in the present invention are the essential oils of ginger and the essential oil piperine. The proportions of essential oils in the additive for animal feed described in the present invention may vary between 1% and 20% of the wet weight of the total product, preferably the proportion is 10% of the final product.

For the purposes of the present invention, the term vegetable or plant fat and/or oil refers to an organic compound obtained from seeds or other parts of plants that accumulate in the tissues as an energy source. The vegetable fats and/or oils used in the present invention to coat the combination of sodium butyrate and the essential oils of ginger and the essential oil piperine comprise palm stearin (the more solid part obtained after fractioning palm oil). The proportions of the vegetable fats and/or oils coating the sodium butyrate and the essential oils of ginger and the essential oil piperine of the present invention may vary between 30% and 60% of the wet weight of the total product, preferably the proportion is 30%.

Furthermore, the present invention describes the use of this additive, natural animal growth promoter, in addition to improving productive parameters of animals (for example increasing their weight), as an immune response modulator and as a bactericide, being able to reduce the incidence in these animals of bacterial diseases such as, for example, necrotic enteritis in poultry, and may also be used for other types of bacterial diseases such as, for example: *Salmonella* Enteritidis, *Escherichia coli, Campylobacter* spp., etc. and in other animals such as, for example: rabbits, pigs, etc.

For the purposes of the present invention, the term bactericide is defined as any product, agent or substance able to remove of kill bacteria. For the purposes of the present invention, the bactericidal effect or action is measured as macroscopic changes produced in the intestines of animals due to bacterial infection. Also, for the purposes of the present invention, modulation of the immune response is measured as the change in gene expression with respect to control animals not receiving the additive of the invention in their feed, infected or not, of genes that code for the cytokines, preferably IL-1β, IL-2, CD3δγ and TNFSF15.

### DESCRIPTION OF THE FIGURES

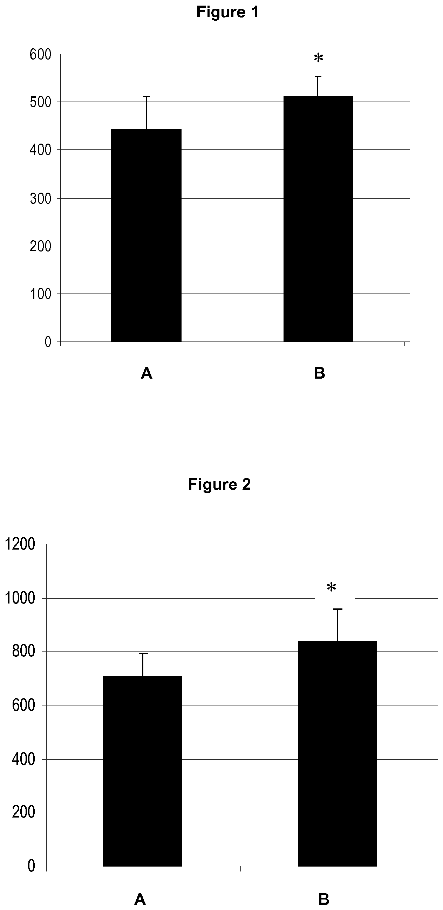

**FIGURE 1****.** Weights of animals of the groups A=control and B=BP-70+ginger and piperine on day 17 of the experimental trial. The Y axis shows the weight in grams. The X axis shows the time in days. * indicates statistically significant differences compared to the infected but untreated control group (p<0.05). BP-70: sodium butyrate (60% of the wet weight) protected with plant oil (30% of the wet weight). The percentage of ginger and piperine included in the additive was 10% of the wet weight of the total product of each of the essential oils that is 5% ginger and 5% piperine.
**FIGURE 2****.** Weights of animals of the groups A=control and B=BP-70+ginger and piperine on day 24 of the experimental trial. The Y axis shows the weight in grams. The X axis shows the time in days. * indicates statistically significant differences compared to the infected but untreated control group (p<0.05). BP-70: sodium butyrate (60% of the wet weight) protected with plant oil (30% of the wet weight). The percentage of ginger and piperine included in the additive was 10% of the wet weight of the total product of each of the essential oils, that is 5% ginger and 5% piperine.

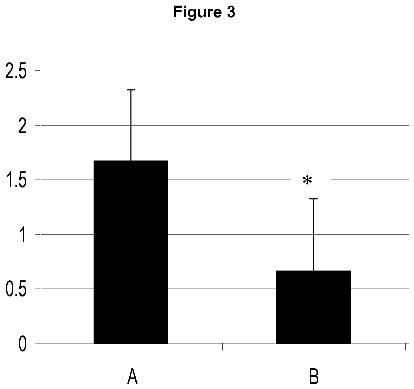

**FIGURE 3****.** Comparison of the macroscopic changes in samples of intestine of the animal group treated with BP-70+ginger and piperine additives (Group B) compared to the macroscopic changes shown by the infected but untreated control animals (Group A). The Y axis shows the score of the macroscopic changes of each group. * indicates statistically significant differences compared to the control group (p<0.05). BP-70: sodium butyrate (60% of the wet weight) protected with plant oil (30% of the wet weight). The percentage of ginger and piperine included in the additive was 10% of the wet weight of the total product, that is 5% ginger and 5% piperine.

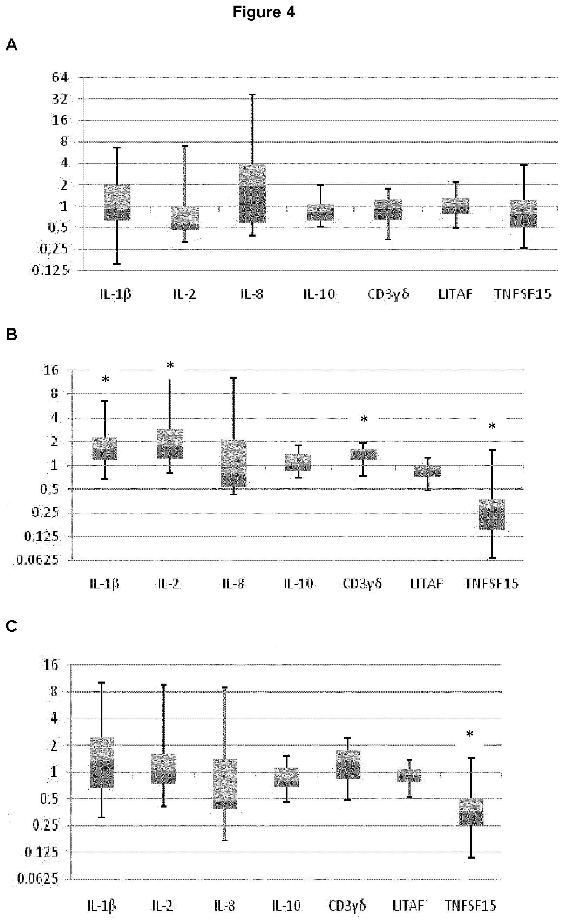

**FIGURE 4****.** Relative levels of the expression (Y axis) of the genes IL-1β, IL-2, IL-8, IL-10, CD3γδ, LITAF and RNFSF15 in the groups of animals treated with BP-70 or BP-70+ginger and piperine compared to the uninfected and untreated animal group (Group A). BP-70: sodium butyrate (70% of the wet weight) protected with plant oil (30% of the wet weight). BP-70+ginger + piperine: sodium butyrate (60% of the wet weight) protected with plant fat (30% of the wet weight) + 5% ginger + 5% piperine. The squared area of the box spans 50% of all measurements, the horizontal line inside the box represents the mean of the sample and the vertical lines represent 50% of the measurements that were outside the values of the box. The asterisks indicate statistical significance with a value of p<0.05. The relative levels of gene expression indicate the expression of each gene compared to the levels of expression of each gene in Group A.
**FIGURE 4A** shows the relative levels of expression of the genes in Group C (uninfected animals treated with BP-70) vs. Group A (uninfected and untreated animals).
**FIGURE 4B** shows the relative levels of gene expression in Group D (uninfected animals treated with BP-70+ginger+piperine) vs. Group A (uninfected and untreated animals).
**FIGURE 4C** shows the relative levels of gene expression in Group F (infected animals treated with BP-70+ginger+piperine) vs. Group A (uninfected and untreated animals).

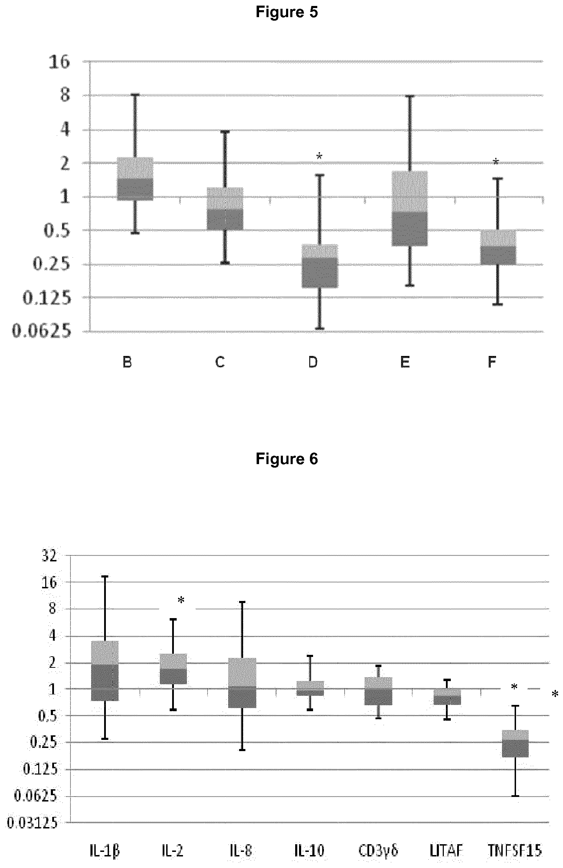

**FIGURE 5****.** Relative levels of expression (Y axis) of the gene TNFSF15 in the various treatment groups: BP-70 (Groups C and E) or BP-70+ginger+piperine (Groups D and F), compared to untreated and uninfected animals (Group A). BP-70: sodium butyrate (70% of the wet weight) protected with vegetable oil (30% of the wet weight). BP-70+ginger + piperine: sodium butyrate (60% of the wet weight) protected with plant fat (30% of the wet weight) + 5% ginger + 5% piperine. Group B: infected animals but not treated; Group C: uninfected animals treated with BP-70: Group D: uninfected animals treated with BP-70+ginger+piperine; Group E: infected animals treated with BP-70; Group F: infected animals treated with BP-70+ginger+piperine. The squared area of the box spans 50% of all measurements, the horizontal line inside the box represents the mean of the sample and the vertical lines represent 50% of the measurements that were outside the values of the box. The asterisks indicate statistical significance with a value of p<0.05. The relative levels of gene expression indicate the expression of each gene compared to the levels of expression of each gene in group a.
**FIGURE 6****.** Relative levels of expression (Y axis) of the genes IL-1β, IL-2, IL-8, IL-10, CD3γδ, LITAF and RNFSF15 in the group of animals infected and treated with BP-70+ginger+piperine (Group F) compared to infected and untreated animals (Group B). BP-70+ginger and piperine: sodium butyrate (60% of the wet weight) protected with vegetable fat (30% of the wet weight) + 5% ginger + 5% piperine. The squared area of the box spans 50% of all measurements, the horizontal line inside the box represents the mean of the sample and the vertical lines represent 50% of the measurements that were outside the values of the box. The asterisks indicate statistical significance with a value of p<0.05. The relative levels of gene expression indicate the expression of each gene compared to the levels of expression of each gene in Group B.

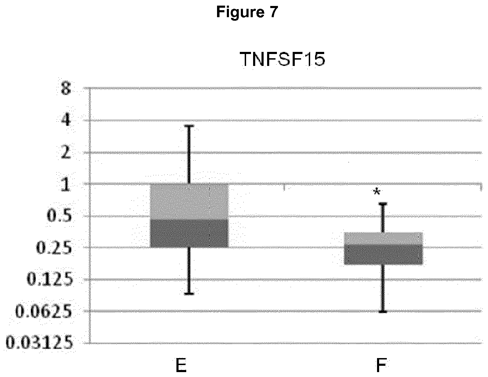

**FIGURE 7****.** Relative levels of expression (Y axis) of the gene TNFSF15 in the groups of animals infected and treated with BP-70 (Group E) or with BP-70+ginger+piperine (Group F) compared to infected and untreated animals (Group B).BP-70: sodium butyrate (70% of the wet weight) protected with plant oil (30% of the wet weight).BP-70+ginger + piperine: sodium butyrate (60% of the wet weight) protected with plant fat (30% of the wet weight) + 5% ginger + 5% piperine. The squared area of the box spans 50% of all measurements, the horizontal line inside the box represents the mean of the sample and the vertical lines represent 50% of the measurements that were outside the values of the box. The asterisks indicate statistical significance with a value of p<0.05. The relative levels of gene expression indicate the expression of each gene compared to the levels of expression of each gene in Group B.

### DETAILED DESCRIPTION OF THE INVENTION

One of the objects of the present invention refers to additives for animal feed comprising the combination of sodium butyrate with the essential oils of ginger and the essential oil piperine together partially coated with vegetable oils and/or fats comprising palm stearin.

The proportions of the sodium butyrate in the additive for animal feed of the present invention may vary between 30% and 70% of the wet weight of the final product, preferably the proportion is 60%.

In another preferred embodiment, the additives of the invention are characterised in that the sodium butyrate is preferably at a concentration of 60% of the wet weight of the final product.

In another preferred embodiment, the additives of the invention are characterised in that the essential oils of ginger and the essential oil piperine are preferably in a proportion of between 1% and 20% of the wet weight of the final product, preferably the proportion is 10% of the wet weight of the final product.

In another preferred embodiment, the additives of the invention are characterised in that the combination of essential oils of ginger and the essential oil piperine is preferably 5% ginger oil and 5% piperine oil.

In another preferred embodiment, the additives of the invention are characterised in that the partial coating of the combination of organic acid salts and active ingredients of plant origin with vegetable oils and/or fats is approximately between 30% and 60% of the wet weight of the final product, preferably the coating is 30%.

In another preferred embodiment, the additives of the invention are characterised in that the vegetable oils and/or fats that partially coat the combination of sodium butyrate and the essential oils of ginger and the essential oil piperine comprise palm stearin.

In another preferred embodiment, the coating of vegetable oils and/or fats is preferably palm stearin.

In another preferred embodiment, the additives of the invention are characterised in that the concentration of sodium butyrate is preferably 60%, the concentration of the combination of the essential oils of ginger and the essential oil piperine is preferably 10% and the coating of palm stearin is preferably 30%.

In another preferred embodiment, the combination of essential oils of ginger and the essential oil piperine is preferably composed of 5% ginger oil and 5% piperine oil.

Another of the objects described in the present invention refers to the use of the additives described above for animal feed, as promoters of animal growth, preferably of chickens, rabbits and pigs.

Another of the objects described in the present invention refers to the use, in animal feed for breeding animals fed with the additives described above, as modulator of the immune response. In another preferred embodiment, the modulation of the immune response comprises a change in the expression of any of the genes that code for any of the following cytokines: increasing the expression of IL-1β, IL-2, CD3γδ and/or reducing the expression of TNFSF15.

Another of the objects described in the present invention refers to the use, in animal feed for breeding animals fed with the additives described above, as a bactericide, inhibiting the growth of both Gram-positive bacteria, preferably bacteria of the genus clostridium, more preferably *C. perfringens,* Gram-negative bacteria, preferably bacteria of the Enterobacteriaceae family, more preferably of the genera *Salmonella, Escherichia* and *Campylobacter* including protozoans such as the case of the protozoans of the *Eimeria* and *Cryptosporidium* families.

The purpose of the examples that are described below is to illustrate the present invention but without limiting its scope.

### EXAMPLE 1. Macroscopic and histological pathological examination in broiler chickens that were induced with necrotic enteritis and treated with various feed additives.

Conventional broiler chickens were used (hybrid Babolna, Babolna, Hungary) that were one day of age when they were distributed to the various treatment groups described in the present invention. Although the examples were carried out in chickens for fattening (poultry farming), the feed additives that potentiate animal growth and with natural bactericidal action, described in the present invention, can be used in any other type of farming such as, for example: pig, rabbit, sheep, cow, etc.

The broiler chickens were fed a conventional diet free of medicines and rich in proteins (25% fishmeal). This feed had no coccidiostats (agents useful in the treatment or prevention of coccidiosis in humans and/or animals). The animals had no restriction to water and were kept in cages at a density of 30 animals/m². The bedding, watering and feeding equipment were previously sterilised in an autoclave. The ambient temperature was initially set at 32 ± 4 °c, reducing to 24 ± 4 °C as the animals grew.

To induce necrotic enteritis, these animals were inoculated with the alpha-toxigenic type a strain of *C. perfringens* (ATCC 13124). This strain was gown in Reinforced Clostridial Medium (BD, MD, USA) culture medium at a temperature of 37 °C for 24 hours in anaerobic conditions making use of the Anaerocult A (Merck, Darmstadt, Germany) system. Then these bacterial cultures were centrifuged (3000 g, 10 min., 5 °C, Universal centrifuge 320R, Hettich centrifuges) and the pellet obtained was resuspended in a solution of sterile phosphate buffered saline (PBS). The *C. perfringens* titre was 3-4x10⁸ colony forming units/ml (CFU/ml). Necrotic enteritis only shows clinical signs if predisposition factors are present. Therefore these animals were infected orally with coccidia by vaccination with the live attenuated vaccine Paracox 5® (Ceva-Phylaxia) and with *C*. *perfringens A.*

Table 1 shows the infection protocol and vaccination used in the present invention. Briefly, the animals were vaccinated against Gumboro disease or nfectious bursitis disease (IBD). This is a highly contagious disease in young chickens caused by infectious bursitis disease virus (IBDV) and is characterised by immunosuppression and death of the animals, usually at the age of between 3 to 6 weeks of age. The vaccine against this disease (CEVAC gumbo L®, Ceva-Phylaxia) was administered in water on day 16 to cause a moderate immunosuppression and to induce the animals to be more susceptible to suffering from necrotic enteritis. Next, they were inoculated orally through a nasogastric tube with 2 mL of a suspension of *C. perfringens* (6.8x10⁸ CFU) three times a day (8:00, 12:00 and 16:00) on days 18, 19, 20 and 21. On day 19, they were also given a dose 10 times higher than normal of the attenuated live vaccine Paracox-5® (Ceva-Phylaxia), to induce infection by coccidia and to predispose the animals to the clinical disease. The Paracox-5® vaccine is an attenuated live vaccine containing sporulated oocysts derived from precocious strains of coccidia that are pathogenic for chickens: *Eimeria acervulina, Eimeria brunetti, Eimeria maxima, Eimeria mitis, Eimeria necatrix, Eimeria praecox and Eimeria tenella.* Control animals were also treated with vaccines against Gumboro disease and with the Paracox-5® vaccine.

**Table 1. Infection and vaccination protocol**

| **Age of chickens (days)** | **1** | **16** | **17** | **18** | **19** | **20** | **21** |
|---|---|---|---|---|---|---|---|
| Vaccination against Gumboro | | | | | | | |
| Infection with Clostridium | | | | | | | |
| Vaccination with Paracox-5® (10 times higher dose than normal) | | | | | | | |

The broiler chickens (n=24) in this present example were divided into three groups (12 animals/group, Table 2). All the birds were treated with vaccines against Gumboro disease and with the Paracox-5® vaccine on the days indicated in Table 1. All the animals of each group were orally infected with *C*. *perfringens A* (6-8x10⁸ CFU). The control group of animals were fed the base diet described, without any additive. In contrast, the other group of animals were fed a diet containing a concentration of 1.5 g/kg feed of the additive described in the present invention: sodium butyrate with ginger and piperine, both active ingredients coated in 30% wet weight of vegetable fats, specifically palm stearin. The sodium butyrate used was the composition from Norel, S.A. called BP-70, to which was added essential oils of ginger (CAS 84696-15-1) (5% wet weight) obtained from Ventós S.A. (Barcelona, Spain) and piperine (5% wet weight) obtained from the company Sensient Fragances S.A. (Granada, Spain). BP-70 is composed of 70% of the wet weight of sodium butyrate and 30% of the wet weight of vegetable fats. The 30% of vegetable fats protects approximately 43% of the total sodium butyrate, therefore the product BP-70 can be described as being composed of 30% protected sodium butyrate and 40% unprotected sodium butyrate. The essential oils of ginger and piperine were coated to the same percentages as the sodium butyrate. All the additives for the feed were supplied by Norel, SA, Spain. The body weight of the animals was measured during the experiment on days 1, 4, 7, 10, 14, 18 and 21. All the animals were sacrificed on day 25.

**Table 2: Experimental design.**

| **Animal groups** | **BP-70 + ginger + piperine (g/kg feed)** | **Paracox-5**® | **Gumboro** | ***C. perfringens* A** |
|---|---|---|---|---|
| **A (n=12)** | - | + | + | + |
| **B (n=12)** | 1.5 g/kg | + | + | + |

| | | | | |
|---|---|---|---|---|
| Grupo A=control, Grupo B=BP-70+ginger+piperine, Group C=essential oils. | | | | |

Figure 1 shows that already on day 17, the day before infection with *C. perfringens* A, the group of animals treated with BP-70+ginger+piperine (Group B) showed an increase in body weight compared to the infected control group but not treated with any feed additive (Group A) (p=0.002). Then another body weight analysis was conducted for each of the groups of animals in the study on day 24, one day before their sacrifice. Figure 2 shows that similarly to the body weight analysis on day 17 (Figure 1), this analysis demonstrated that the group treated with BP-70+ginger+piperine showed a significantly higher body weight gain than the control animal group that were infected but not treated with any feed additive (p=0.001).

Next, a histopathological analysis was conducted of the samples collected from various organs of each group of animals: liver, spleen and distal jejunum. Fresh samples were immediately submerged in 10% formalin for preservation until they were used in subsequent analyses. Microscopic lesions were classified in accordance with the method of Gholamiandehkordi et al (2007) with small deviations: 0=negative; 1=inflammation of the intestine; 2=focal necrosis (1.2 cm) or degenerative changes in the mucosa (fatty appearance); 3=irregular necrosis (3-4 cm) and 4=diffuse necrosis. The presence of enteritis (generally histiocytic lymphoma) was also noted.

Figure 3 shows a comparison of the severity of the macroscopic lesions seen in the group of animals treated with BP-70+ginger+piperine (Group B) compared to the control group of animals infected but not treated with any additive. As shown in Figure 3, Group B (BP-70+ginger+piperine) showed significantly lower scores than the control group (p=0.001). Therefore, these results demonstrate that treatment with BP-70+ginger+piperine (Group B) significantly reduced macroscopic legions found in broiler chickens infected with *C. perfringens A* compared to control animals that showed infection but were not administrated with any type of additive (Group A).

Thus the present example demonstrates that treatment with the feed additive described in the present invention, sodium butyrate and plant essential oils, ginger and piperine, together partially coated with vegetable fats, produces a significant increase in the weight of the animals with artificial necrotic enteritis induced by inoculation with *C. perfringens A* and treated with this additive (Group B) compared to the control animal group, which also showed signs of infection but were not treated with this additive (Group A). This example also demonstrates the improvement in the lesions, mainly intestinal (caused by infection with *C. perfringens A*), that the samples extracted from the group of animals treated with the additive of the invention (Group B) showed compared to the control animal group that were not treated with any additive (Group A).

### Example 2. Determination of the immune response of gut-associated lymphoid tissue (GALT) in broiler chickens suffering from necrotic enteritis and treated with various feed additives.

In order to determine the immune response of the GALT, another experimental trial was carried out with broiler chickens (n=48) that were divided into six groups (6 animals/group, Table 3). All the birds, similarly to Example 1, were treated with vaccines of Gumboro and Paracox-5® on all the days indicated in Table 1. The groups A, C and D were composed of broiler chickens not infected with *C. perfringens A,* whereas groups B, E and F were composed of broiler chickens orally infected with *C*. *perfringens A* (6-8x10⁸ CFU). The base diet of the animals was the same as described in Example 1, but complemented with various additives for feed: (i) 1.5 g of BP-70/kg. of feed (Groups C and E), (ii) 1.5 g of BP-70+ginger+piperine/kg feed (Groups D and F). All the additives for feed were supplied by Norel, SA (Madrid, Spain).

**Table 3. Experimental design**

| **Group** | **BP-70 (g/kg feed)** | **BP-70+ginger + piperine: (g/kg feed)** | **Paracox-5**® **vaccine** | **Gumboro vaccine** | ***Clostridium perfringens* A** |
|---|---|---|---|---|---|
| **A** | - | - | + | + | - |
| **B** | - | - | + | + | + |
| **C** | 1.5 g/kg | - | + | + | - |
| **D** | - | 1.5 g/kg | + | + | - |
| **E** | 1.5 g/kg | - | + | + | + |
| **F** | - | 1.5 g/kg | + | + | + |

The chickens were sacrificed on day 22 and intestinal samples were taken of approximately 5 cm length of the jejunum between Meckel's diverticulum and the iliac region. The intestinal sections were cut longitudinally and were washed three times with iced PBS containing 100 U/ml penicillin and 100 µg/ml streptomycin (Fischer Scientific). The mucous layer was scraped with a sterile glass slide and the intestinal tissue was immediately submerged in 1 ml ice cold TRizol (Invitrogen) for subsequent extraction of RNA according to the manufacturer's instructions. The samples were homogenised and maintained at -80 °C until processing.

In order to evaluate the inflammatory and immune response after inducing infection with necrotic enteritis in the animals and comparing the effects of each of the treatments with the various additives described in Table 3, the changes in the levels of expression of various cytokines was evaluated by the technique of quantitative PCR (qRT-PCT). The cytokines analysed were: interleukin-1 beta (IL-1β), interleukin-2 (IL-2), interleukin-8 (IL-8), interleukin-10 (IL-10), CD3γδ receptor, lipopolysaccharide-induced tumour necrosis factor-alpha factor (LITAF) and factor 15 of the super-family of the tumour necrosis factor (TNFSF15). The control genes used as reference were: glyceraldehyde-3-phosphate dehydrogenase (GAPDH) and β-actin. The primers used for each of the genes mentioned are shown in Table 4. The interleukins-1, 2, 8 and 10 are members of the interleukin family, essential for the cellular and humoral immune response. The CD3 receptor (cluster of differentiation 3) is a target receptor found in all T-cells, LITAF is a cytokine of the TNF family that is released after LPS induction and TNFSF15 is present in endothelial cells and also participates in proliferation, differentiation and apoptosis of the cells of the immune system.

In order to analyse significant differences shown by each of the treatment groups described above in the levels of expression of the genes analysed, the ANOVA test was used.

**Table 4. Primers used in the qRT-PCT technique**

| RNA | Primers | | Product size | GenBank Accession No. |
|---|---|---|---|---|
| Gene | Direct | Inverse | (bp) | |
| GAPDH | SEQ ID No. 1 | SEQ ID No. 2 | 264 | NM_204305 |
| β-actin | SEQ ID No. 3 | SEQ ID No. 4 | 169 | NM_205518 |
| IL-1β | SEQ ID No. 5 | SEQ ID No. 6 | 80 | NM_204524 |
| IL-2 | SEQ ID No. 7 | SEQ ID No. 8 | 148 | NM_204153 |
| IL-8 | SEQ ID No. 9 | SEQ ID No. 10 | 200 | NM_205498 |
| IL-10 | SEQ ID No. 11 | SEQ ID No. 12 | 94 | NM_001004414 |
| CD3γδ | SEQ ID No. 13 | SEQ ID No. 14 | 166 | NM_205512.1 |
| LITAF | SEQ ID No. 15 | SEQ ID No. 16 | 229 | NM_204267 |
| TNFSF15 | SEQ ID No. 17 | SEQ ID No. 18 | 292 | NM_001024578 |

In order to demonstrate the immunomodulator effects provided by the feed additives administered to the various animal groups described in the present example, either infected or not with *C. perfringens,* animal Groups C and D that were treated with some of the additives mentioned and that did not show infection were compared to the untreated and uninfected control group of animals (Group A). Also, the animal groups E and F that were treated with the additives BP-70 or BP-70)+ginger+piperine respectively and that showed necrotic enteritis were compared against the group of animals infected with *C. perfringens* but not treated with any additive of those described in the present invention (Group B).

Firstly, the levels of expression of the cytokines analysed in the untreated control group of animals that were not infected with *C. perfringens A* (Group A) were compared to the groups of animals not infected with *C. perfringens A* but treated with the feed additives BP-70 (Group C) and BP-70+ginger+piperine (Group D) to analyse the natural immunomodulator effects of these additives.

Figures 4A, B and C show the immunomodulator effect of the additives: BP-70 and BP-70+ginger+piperine on the level of expression of the genes IL-1β, IL-2, IL-8, IL-10, CD3γδ, LITAF and TNFSF15. The relative expression ratios of these genes in the treated groups C and E were compared to those in untreated animals (Group A). None of the groups were infected with *C. perfringens A.* Figure 4A shows that treatment with BP-70 (Group C) did not cause any change in the expression levels of the genes analysed. The major changes in the expression profile of the genes analysed occurred due to the treatment with the additive BP-70+ginger+piperine (Group D) (Figure 4B). Three of the genes investigated were over expressed in the group of animals taking BP-70+ginger+piperine (Group D) compared to the untreated control group of animals (Group A). These genes were: IL-1β (p=0.028), IL-2 (p=0.026) and CD3γδ (p=0.011). One of the genes analysed, TNFSF15, showed underexpression (p=0.004). This underexpression was also reproduced (Figure 4C) when comparing the untreated and uninfected control group of animals (Group A) to the infected group of animals treated with the additive described in the present invention, BP-70+ginger+piperine (Group F). Overexpression of the genes IL-1β and IL-2 indicates the activation of the immune response cascade, giving rise to stimulation of proliferation and maturation of lymphocytes. These observations were confirmed by the increase in gene expression of CD3γδ that represents a raised total T-cell count.

Figure 5 also shows the levels of expression of each of the cytokines analysed: IL-1β, IL-2, IL-8, IL-10, CD3γδ, LITAF and TNFSF15, in each of the groups of animals treated with each of the additives described in the present example and that were either infected or not with *C. perfringens A* (Groups C to F), as well as the control group of animals infected with *C. perfringens A,* but not treated with any of these additives (Group B). Figure 5 demonstrates that treatment with BP-70+ginger+piperine (Group E) caused underexpression of the gene TNFSF15 compared to both the control and uninfected group of animals that were not given any additive in their diet (Group A) and in the infected group of animals that were also not given any additive in their diet (Group B).

Secondly, the immunomodulator effects provided by feed additives given to animals of the groups E (BP-70) and F (BP-70+ginger+piperine), treated with the additives mentioned and that had necrotic enteritis, were analysed with respect to the group of animals infected with *C. perfringens* but not treated with any additive of those described in the present invention (Group B).

Figure 6 shows the levels of expression of the genes IL-1β, IL-2, IL-8, IL-10, CD3γδ, LITAF and TNFSF15 in the group of animals infected with *C. perfringens A* and that were treated with the feed additive described in the present invention, BP-70+ginger+piperine (Group F), compared to the levels of expression of these genes in the group of control animals infected with *C. perfringens A* but that were not fed with the additives used in the present example (Group B). This analysis was carried out to analyse the effects of these additives in the evolution of the infection. Figure 6 shows that there was a significant increase in the levels of expression of cytokine IL-2 compared to the control untreated group (Group B). Furthermore, the group of animals treated with BP-70+ginger+piperine showed a decrease in the levels of expression of TNFSF15 compared to the untreated control group of animals (Group B).

Similarly, Figure 7 shows the level of expression of cytokine TNFSF15 in the group of animals infected and treated with BP-70+ginger+piperine (Group F) compared to the control group of animals that were infected but not treated (Group B). Treatment with BP-70+ginger+piperine (Group F) caused underexpression of TNFSF15 compared to the control group (Group B).

In accordance with the results shown in the present example, treatment with sodium butyrate + essential oils, exemplified in the present invention by treatment with BP-70+ginger+piperine, was able to show a significant modulator action on the immune response, causing an increase in the expression of genes coding for the cytokines IL-1β, IL-2 and CD3γδ and a decrease in the expression of the gene coding for the cytokine TNFSF15, giving rise to a protector effect against artificial infection caused by *C. perfringens A.*

Furthermore, as demonstrated in the results shown in Example 1, treatment with the additive described in the present invention is also able to induce an increase in weight gain of animals subjected to this treatment and reduce the severity of histological lesions shown by these animals as a consequence of infection with *C. perfringens A.*

### BIBLIOGRAPHY

1. Dipeolu et al. Biocatalytic amide reduction using Clostridium sporogenes. Biotechnol Lett. 2005 Nov;27(22):1803-7.
2. Fernandez-Rubio C, Ordóñez C, Abad-González J, Garcia-Gallego A, Honrubia MP, Mallo JJ, Balaña-Fouce R. Butyric acid-based feed additives help protect broiler chickens from Salmonella Enteritidis infection. Poult Sci. 2009 May;88(5):943-8.
3. Gálfi P, Bokori J. Feeding trial in pigs with a diet containing sodium n-butyrate. Acta Vet Hung. 1990;38(1-2):3-17.
4. Gholamiandehkordi AR, Timbermont L, Lanckriet A, Van Den Broeck W, Pedersen K, Dewulf J, Pasmans F, Haesebrouck F, Ducatelle R, Van Immerseel F. Quantification of gut lesions in a subclinical necrotic enteritis model. Avian Pathol. 2007 Oct;36(5):375-82.
5. Katoh K y Tsuda T, Effects of acetylcholine and short-chain fatty acids on acinar cells of the exocrine pancreas in sheep. J Physiol. 1984 Nov;356:479-89.
6. Leeson S, Namkung H, Antongiovanni M, Lee EH. Effect of butyric acid on the performance and carcass yield of broiler chickens. Poult Sci. 2005 Sep;84(9):1418-22.
7. Sano H, Nakamura E, Takahashi H, Terashima Y. Plasma insulin and glucagon responses to acute challenges of acetate, propionate, n-butyrate and glucose in growing gilts (Sus scrofa). Comp Biochem Physiol A Physiol. 1995 Apr;110(4):375-8.
8. Van Immerseel, F., V. Fievez, J. De Buck, F. Pasmans, A. Martel, F. Haesebrouck, and R. Ducatelle. Microencapsulated short-chain fatty acids in feed modify colonization and invasion early after infection with Salmonella Enteritidis in young chickens. Poult. Sci. 2004, 83:69-74.

## Claims

1. Additive for animal feed comprising the combination of sodium butyrate with the essential oils of ginger and the essential oil piperine, wherein said combination is partially coated with vegetable oils and/or fats and wherein the vegetable oils and/or fats comprise palm stearin.

2. Additive for animal feed according to claim 1, wherein the sodium butyrate is in a proportion of 30-70% of the wet weight.

3. Additive for animal feed according to claim 1 or 2, wherein the essential oils of ginger and the essential oil piperine, are in a concentration of 1-20% of the wet weight.

4. Additive for animal feed according to any one of claims 1-3, wherein the essential oils of ginger are in a concentration of 5% of the wet weight and the essential oil piperine is in a concentration of 5% of the wet weight.

5. Additive for animal feed according to claim 1, wherein the partial coating with vegetable oils and/or fats comprising palm stearin is 30-60% wet weight.

6. Additive for animal feed according to any one of claims 1-5, wherein the concentration of sodium butyrate is 60% of the wet weight, the concentration of the essential oils of ginger and the essential oil piperine is 10% of the wet weight and wherein the partial coating is a palm stearin coating and the concentration of the palm stearin coating is 30% of the wet weight.

7. The additive for animal feed according to any one of claims 1-6 for use as animal growth promoter.

8. The additive for animal feed according to any one of claims 1-6 for use as modulator of the immune response.

9. The additive for animal feed for use according to claim 8, wherein the modulation of the immune response comprises a change in the expression of any of the genes coding for any of the following cytokines: increasing the expression of IL-1β, IL-2 CD3γδ and/or reducing the expression of TNFSF15.

10. The additive for animal feed according to any one of claims 1-6 for use as bactericide.

11. The additive for animal feed for use according to claim 10, wherein the bactericidal action is exercised on Gram-positive and Gram-negative bacteria and on protozoans.

## Patentansprüche

1. Additiv für Tierfutter umfassend die Kombination von Natriumbutyrat mit den ätherischen Ölen von Ingwer und dem ätherischen Öl Piperin, worin die Kombination partiell mit Pflanzenölen und/oder Fetten beschichtet ist und worin die Pflanzenöle und/oder Fette Palmstearin umfassen.

2. Additiv für Tierfutter nach Anspruch 1, worin das Natriumbutyrat zu einem Anteil von 30-70% des Nassgewichts vorhanden ist.

3. Additiv für Tierfutter nach Anspruch 1 oder 2, worin die ätherischen Öle von Ingwer und das ätherische Öl Piperin in einer Konzentration von 1-20% des Nassgewichts vorhanden sind.

4. Additiv für Tierfutter nach einem der Ansprüche 1-3, worin die ätherischen Öle von Ingwer in einer Konzentration von 5% des Nassgewichts und das ätherische Öl Piperin in einer Konzentration von 5% des Nassgewichts vorhanden ist.

5. Additiv für Tierfutter nach Anspruch 1, worin die Palmstearin umfassende partielle Beschichtung mit Pflanzenölen und/oder Fetten 30-60% des Nassgewichts beträgt.

6. Additiv für Tierfutter nach einem der Ansprüche 1-5, worin die Konzentration von Natriumbutyrat 60% des Nassgewichts beträgt, die Konzentration der ätherischen Öle von Ingwer und des ätherischen Öls Piperin 10% des Nassgewichts beträgt und worin die partielle Beschichtung eine Palmstearinbeschichtung ist und die Konzentration der Palmstearinbeschichtung 30% des Nassgewichts beträgt.

7. Additiv für Tierfutter nach einem der Ansprüche 1-6 zur Verwendung als Tierwachstumsförderer.

8. Additiv für Tierfutter nach einem der Ansprüche 1-6 zur Verwendung als Modulator der Immunantwort.

9. Additiv für Tierfutter nach Anspruch 8, worin die Modulation der Immunantwort eine Änderung der Expression eines der Gene umfasst, die für eines der folgenden Zytokine kodieren: Erhöhung der Expression von IL-1β, IL-2CD3γδ und/oder Reduzierung der Expression von TNFSF15.

10. Additiv für Tierfutter nach einem der Ansprüche 1-6 zur Verwendung als Bakterizid.

11. Additiv für Tierfutter nach Anspruch 10, worin die bakterizide Wirkung auf grampositive und gramnegative Bakterien und auf Protozoen ausgeübt wird.

## Revendications

1. Additif pour aliments destinés à l'alimentation animale comprenant la combinaison de butyrate de sodium avec les huiles essentielles de gingembre et l'huile essentielle pipérine, dans lequel ladite combinaison est partiellement enrobée d'huiles végétales et/ou graisses et dans lequel les huiles végétales et/ou graisses comprennent de la stéarine de palme.

2. Additif pour aliments destinés à l'alimentation animale selon la revendication 1, dans lequel le butyrate de sodium est présent dans une proportion de 30-70% du poids humide.

3. Additif pour aliments destinés à l'alimentation animale selon la revendication 1 ou 2, dans lequel les huiles essentielles de gingembre et l'huile essentielle pipérine, sont présentes à une concentration de 1-20% du poids humide.

4. Additif pour aliments destinés à l'alimentation animale selon l'une quelconque des revendications 1-3, dans lequel les huiles essentielles de gingembre sont présentes à une concentration de 5% du poids humide et l'huile essentielle pipérine est présente à une concentration de 5% du poids humide.

5. Additif pour aliments destinés à l'alimentation animale selon la revendication 1, dans lequel l'enrobage partiel avec des huiles végétales et/ou graisses comprenant de la stéarine de palme est de 30-60% du poids humide.

6. Additif pour aliments destinés à l'alimentation animale selon l'une quelconque des revendications 1-5, dans lequel la concentration de butyrate de sodium est de 60% du poids humide, la concentration des huiles essentielles de gingembre et de l'huile essentielle pipérine est de 10% du poids humide et dans lequel l'enrobage partiel est un enrobage de stéarine de palme et la concentration de l'enrobage de stéarine de palme est de 30% du poids humide.

7. Additif pour aliments destinés à l'alimentation animale selon l'une quelconque des revendications 1-6 à utiliser comme stimulateur de croissance pour animaux.

8. Additif pour aliments destinés à l'alimentation animale selon l'une quelconque des revendications 1-6 à utiliser comme modulateur de la réaction immunitaire.

9. L'additif pour aliments destinés à l'alimentation animale à utiliser selon la revendication 8, dans lequel la modulation de la réaction immunitaire comprend un changement dans l'expression de l'un quelconque des gènes codant pour l'une quelconque des cytokines suivantes : augmentation de l'expression de IL-1β, IL-2CD3γδ et/ou diminution de l'expression de TNFSF15.

10. L'additif pour aliments destinés à l'alimentation animale selon l'une quelconque des revendications 1-6 à utiliser comme bactéricide.

11. L'additif pour aliments destinés à l'alimentation animale à utiliser selon la revendication 10, dans lequel l'action bactéricide est exercée sur des bactéries à Gram positif et à Gram négatif et sur des protozoaires.
